# EUROPEAN PATENT SPECIFICATION

(11) **EP 3 025 659 B1**
(45) Date of publication and mention of the grant of the patent: **30.12.2020**
(21) Application number: 14828953.1
(22) Date of filing: 10.06.2014
(51) Int. Cl.: A61B 17/16, A61B 17/32

(54) **PIEZOSURGERY TOOL BIT AND PIEZOSURGERY TOOL HAVING SAME**
PIEZOCHIRURGISCHER WERKZEUGHALTER UND PIEZOCHIRURGISCHES WERKZEUG DAMIT
INSERT D'OUTIL DE PIÉZOCHIRURGIE ET PIÉZOCHIRURGIE EMPLOYANT CET INSERT D'OUTIL

(30) Priority: 22.07.2013 CN 201320435087 U
(43) Date of publication of application: 01.06.2016
(73) Proprietor: Cao, Qun, Beijing 100081 (CN); Li, Chunyuan, Beijing 100081 (CN)
(72) Inventor: Cao, Qun, Beijing 100081 (CN); Li, Chunyuan, Beijing 100081 (CN)
(74) Representative: Vigand, Philippe
(86) International application number: PCT/CN2014/079578
(87) International publication number: WO 2015/010505

(56) References cited:
- EP-A1- 0 456 470
- EP-A1- 1 736 107
- WO-A1-2014/088899
- CN-A- 1 745 721
- CN-A- 102 143 715
- CN-A- 103 431 894
- CN-U- 202 146 339
- CN-U- 202 821 533
- CN-U- 202 821 533
- CN-U- 203 354 612
- CN-Y- 200 942 107
- CN-Y- 201 404 264
- US-A- 5 318 570
- US-A- 5 695 510
- US-A1- 2010 057 118
- US-A1- 2011 270 256
- US-A1- 2013 184 711

## Description

### BACKGROUND OF THE INVENTION

### 1. Field of the Invention

The present disclosure relates to a technical field of medical equipment, in particular to an improved tool bit of ultrasonicosteotome and an ultrasonicosteotome comprising the tool bit.

### 2. Description of the Related Art

Conventional orthopedicequipments such as an electric drill and ultrasonicosteotome apply downward forces in most of operations in surgery, which may bring great risk.

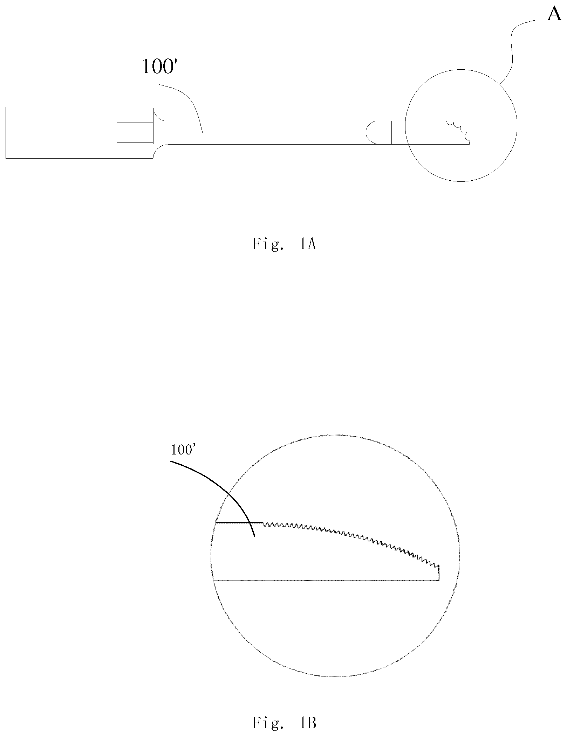

Fig. 1A shows a typical structure of a conventional tool bit 100' of ultrasonicosteotome, which has a plurality of teeth and a wide tip. However, a majority part of the tip does not constitute an effective main cutting part, as shown in Fig. 1B.

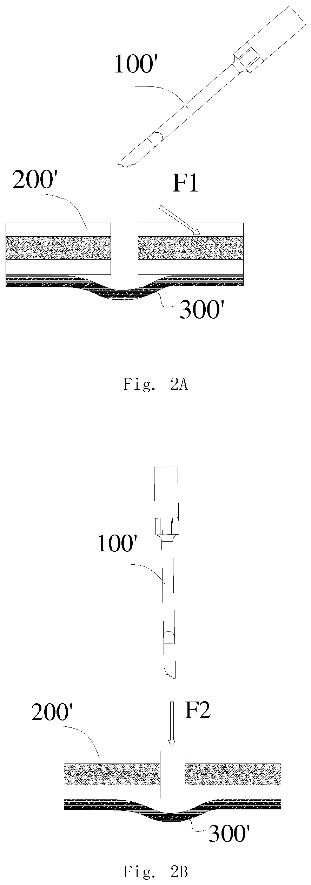

The tool bit 100' also has the following defects. As illustrated in Fig. 2A, when the tool bit 100' is used, a force needs to be applied to osseous tissue of a bone 200', as indicated by F1 in Fig. 2A. When the cutting operation is desired, the tool bit 100' needs to apply the force downwardly to the osseous tissue, which may not only cause a low cutting speed and a low efficiency, but also cause the tool bit with a plurality of teeth to break in operation, as indicated by F2 in Fig. 2B. Further, as illustrated in Figs. 2A and 2B, soft tissue 300' below the cutting location is invisible. As the force is applied downwardly in operation, it tends to destroy the tissues which are located below the cutting location and are undesired to be injured. In this way, the risk of surgery may be increased significantly, chance of success in surgery may be reduced, and operation difficulty of a doctor may be increased. For example, in a backbone surgery, typically, the spinal cord is just located below the surgical site for the backbone surgery. If the spinal cord is injured, the patient will at least lose a function, be permanently paralysed, even decease.

As the relevant patent documents of this application, US2013/184711A1 discloses a sonotrode for an ultrasonic surgical instrument , US5695510A discloses a blade for ultrasonic surgery which comprises a generally planar body having at least one cutting edge thereon and a longitudinal axis, US2010/057118A1 discloses an ultrasonic surgical blade comprising a body having an external surface, a distal end movable relative to a longitudinal axis by vibrations applied thereto, and a cutting edge having a distal portion and a proximal portion , CN202146339 discloses a double-circular arc-shaped tool bit of an ultrasonicosteotome comprising a blade and a tool nose, CN202821533 discloses a tool bit of an ultrasonicosteotome comprising an arbor and a head end, the head end is arranged at the front end of the arbor and the head end is spoon-like shape. For these ultrasonic surgical instruments, neither can improve the accuracy of position nor can balance the cutting efficiency and safety in surgery.

EP1736107A1 discloses a tip for a bone microsurgery of the type that comprises at least one coupling, an actuation handpiece, wherein one head of said actuation handpiece has at least one of the lateral sides, a distribution of sharp teeth having a preset thickness, height and width. As described in paragraphs 27-30 of EP1736107A1, the tip is particularly suitable for use in association with a handpiece of the type with pneumatic-mechanical actuation, not for use in association with a handpiece of the type with supersonic actuation.

### SUMMARY

In order to solve at least one of the above problems in the prior art, the present disclosure provides a tool bit of an ultrasonicosteotome that has a high efficiency and a high cutting speed to reduce the time required for surgery.

Further, the present disclosure provides a tool bit of an ultrasonicosteotome that may be positioned correctly in cutting operation to improve the chance of success in surgery.

Further, the present disclosure also provides an ultrasonicosteotome including the tool bit as described above.

In accordance with the invention as defined by claim 1, a tool bit of an ultrasonic osteotome comprises: an arbor; and an end portion of the tool bit connected to a front end of the arbor, the end portion of the tool bit has a spoon-like shape wherein the end portion of the tool bit has a toothed front edge,
wherein a front edge of the end portion of the tool bit is formed with a plurality of tooth slots substantially extending in a direction from a concave surface of the end portion of the tool bit to a convex surface thereof; and
wherein each of the tooth slots has an end in communication with the concave surface of the end portion of the tool bit, and has a length reduced gradually from a central part one of the tooth slots to two lateral part ones of the tooth slots in a width direction of the end portion of the tool bit.

The tool bit according to the present invention, may increase the cutting speed to reduce the time required for surgery, and may be positioned correctly in cutting operation without skidding to improve the chance of success in surgery.

In addition, the tool bit according to an embodiment of the present invention may also comprise the following additional technical features:
In an embodiment, the front edge of the end portion of the tool bit has a maximum width not less than the width of the arbor.

In an embodiment, both the arbor and the end portion of the tool bit have hollow inner chambers that are communicated with each other.

In an embodiment, the end portion of the tool bit is formed with a bore which is located in at least one of the spoon center and the spoon bottom of the end portion of the tool bit and is in communication with the hollow inner chambers.

In an embodiment, the tool bit further comprises:a bit body connected to a rear end of the arbor, wherein the arbor and the bit body are of cylindrical shape, the bit body has a cylindrical dimension greater than that of the arbor, and the bit body and the arbor are connected via an arc transition.

In an embodiment, the bit body is provided with a wrench-operation position.

In an embodiment, a screwed connection hole is formed on the side of the bit body opposed to the arbor and is communicated with the hollow inner chambers.

In an embodiment, each of the tooth slots is at an angle of less than 90 degrees with respect to the direction from the concave surface of the end portion of the tool bit to the convex surface thereof.

In an embodiment, at least one of two opposed side walls of the end portion of the tool bit connected to the convex surface and the concave surface respectively is provided with a plurality of first grooves spaced from each other.

Preferably, each of the first grooves extends in a direction from a spoon center to a spoon bottom and the plurality of first grooves are distributed in a direction from the front to the rear.

In some embodiments, a convex surface of the end portion of the tool bit is provided with a plurality of second grooves.

Preferably, each of the second grooves extends in the width direction of the end portion of the tool bit and the plurality of second grooves are distributed in a direction from the front to the rear.

An embodiment of the present invention as defined by claim 13 also provides an ultrasonic osteotome comprising the tool bit according to any one of the above embodiments.

Such design of the ultrasonic osteotome according to the embodiment of the present invention, not only may increase the cutting speed to reduce the time required for surgery, but also may be positioned correctly in cutting operation without skidding to improve the chance of success in surgery.

### BRIEF DESCRIPTION OF THE DRAWINGS

The above and/or additional aspects and advantages of the present disclosure will be understood more apparently from the following description of embodiments with reference to the accompanied figures, in which:
Fig. 1A is a schematic view showing a structure of a tool bit of an ultrasonic osteotome in the prior art;
Fig. 1B is a local enlarged schematic view of part A shown in Fig. 1A;
Figs. 2A-2B are schematic views showing operations of the tool bit of the ultrasonic osteotome in the prior art;
Fig. 3 is a schematic view showing a perspective structure of a tool bit of an ultrasonic osteotome in accordance with an embodiment of the present invention;
Fig. 4 is a top view of the tool bit of the ultrasonic osteotome in accordance with an embodiment of the present invention;
Fig. 5 is a front view of the tool bit of the ultrasonic osteotome in accordance with an embodiment of the present invention;
Fig. 6 is a schematic view showing a local enlarged structure of part B shown in Fig. 3;
Fig. 7 is a schematic view showing a local enlarged structure of part B shown in Fig. 3, according to another embodiment;
Fig. 8 is a schematic view showing the tool bit of the ultrasonic osteotome in a usage state in accordance with an embodiment of the present invention; and
Fig. 9 is a schematic view showing the tool bit of the ultrasonic osteotome in another usage state in accordance with an embodiment of the present invention.

### DETAILED DESCRIPTION OF THE EMBODIMENTS

The specific embodiments will be described in detail below with reference to accompanied figures. Throughout the description, identical or similar reference numerals represent identical or similar members. It should be noted that embodiments described herein are depicted only for illustration, instead of limiting to the present invention.

In the description of the present disclosure, it should be noted that the orientations or positions represented by the terms of "up", "down", "vertical", "horizontal", "top", "bottom", "inner" and "outer" and the like are based on the figures. It is given only by way of examples, instead of being intended to limit the device or element to have a special orientation or to be configured and operated in a special orientation. Thus, it cannot be understood as limiting of the present invention. In addition, the terms of "first" and "second" are depicted only by illustration, instead of being intended to define which one is more important or to define the number of the technical features. Thereby, the features defined by the terms of "first" and "second" may literally or impliedly include one or more features. In the description of the present disclosure, the term of "a plurality of" means two or more, unless it is explained otherwise.

In the description of the present disclosure, it should be noted that the terms of "mount", "connect" and "couple" should be understood in broad sense, unless they are defined or provided otherwise. For example, they may be used to describe a fixed connection, or a dismountable connection or an integral connection; they may be used to describe a mechanical connection, an electrical connection; they may be used to describe direct connection or connection by intermediate medium, or communication between interiors of two elements. The specific significations of the above terms in the present disclosure may be understood in the context by the skilled person in the art.

The technical solutions of the present disclosure will be further explained below with reference to figures and embodiments.

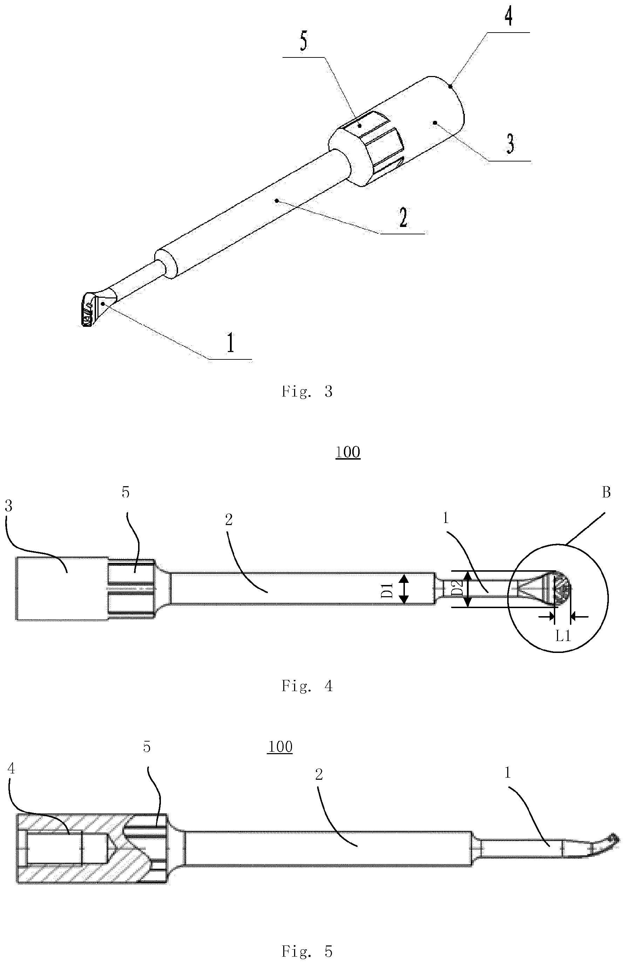

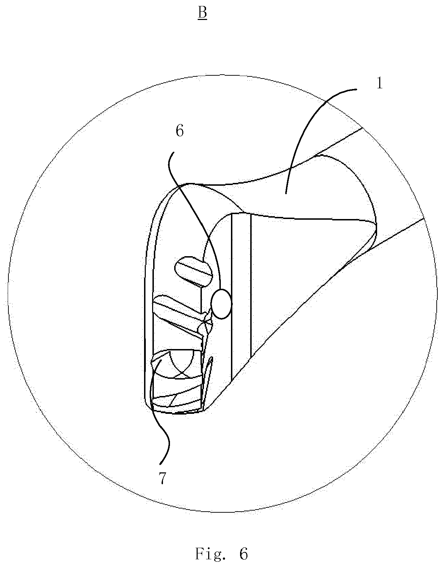

A tool bit of an ultrasonic osteotome in accordance with an embodiment of the present invention will be described below with reference to figures. Fig. 3 is a schematic view showing a perspective structure of a tool bit 100 of an ultrasonic osteotome in accordance with an embodiment of the present invention. Fig. 4 is a top view of the tool bit 100 of the ultrasonic osteotome in accordance with an embodiment of the present invention. Fig. 5 is a front view of the tool bit 100 of the ultrasonic osteotome in accordance with an embodiment of the present invention. Fig. 6 is a schematic view showing a local enlarged structure of part B shown in Fig. 3

As illustrated in Fig. 3, in accordance with an embodiment of the present invention, the tool bit 100 of the ultrasonic osteotome includes an end portion 1 of the tool bit and an arbor 2. The end portion 1 of the tool bit is connected to a front end of the arbor 2. The end portion 1 of the tool bit has a spoon-like shape and has a toothed front edge. Such design may increase the cutting speed to reduce the time required for surgery. Further, it may be positioned correctly in cutting operation without skidding, so as to improve the chance of success in surgery.

As illustrated in Fig. 3, a bit body 3 is connected to the arbor 2. The arbor 2 is a thick cylindrical section while the bit body 3 is a thin cylindrical section. The bit body 3 and the arbor 2 are connected via an arc transition. The bit body 3 is provided with a wrench-operation position 5 thereon. A threaded connection hole 4 is formed on the side of the bit body 3 opposed to the arbor 2. The threaded connection hole 4 is configured to be connected in thread with an ultrasonic transducer (not shown). When it needs to be used, the threaded connection hole 4 at a rear end of the tool bit is connected to a special ultrasonic transducer, and the connection is tighten by a corresponding wrench at the wrench-operation position 5. And then the ultrasonic transducer is connected to a special ultrasonic machine and thereby an ultrasonic cutting may be available.

In accordance with an embodiment of the present invention, the front edge of the end portion 1 of the tool bit 100 has a maximum width D2 being not less than the width D1 of the arbor 2. Such structure has an advantage that the cutting width of the end portion 1 of the tool bit is greater than or equal to the diameter of the arbor 2 in use. In surgery, when a thicker bone or a deeper groove or window-type incision is cut, it may prevent the tool bit from being jammed while ensuring water to pour to the cutting position of the end portion of the tool bit, so as to prevent the incision from being burned.

In accordance with an embodiment of the present invention, both the arbor 2 and the end portion 1 of the tool bit 100 have hollow inner chambers that are communicated with each other. The end portion 1 of the tool bit has a bore which is located in the spoon center. Such design may have an advantage that the tool bit with single outlet at the spoon center may ensure the poured liquid to pass through the hollow chamber to the spoon center and to flow out of the tool bit. In this way, in surgery, the spoon center, the entire end portion 1 of the tool bit and the tissue they contact with may be cleaned and cooled by the poured liquid in real time completely, so as to ensure the removed bone crumbs to be discharged timely. Thus, there is a clear and clean view at the incision and the tissue to be remained is protected from being damaged, which is particularly noticeable in scraping operation. In this way, the surgery risk will be further reduced, and the safety together with the chance of success in surgery will be improved.

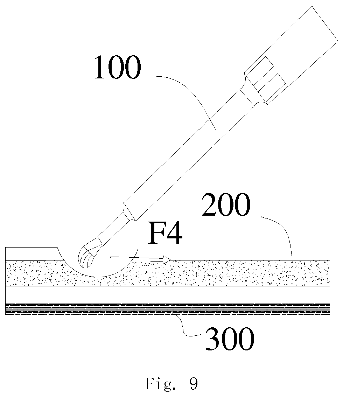

In accordance with an embodiment of the present invention, both the arbor 2 and the end portion 1 of the tool bit 100 have hollow inner chambers that are communicated with each other. The bore 6 of the end portion 1 of the tool bit may be located in the spoon center, i.e., the bore 6 is arranged on the concave surface, as illustrated in Fig. 6. Such design may have an advantage that the tool bit with single outlet at the spoon center may ensure the poured liquid to pass through the hollow chamber to the spoon center and to flow out of the tool bit. In this way, in surgery, the spoon center, the entire end portion 1 of the tool bit and the tissue they contact with may be cleaned and cooled by the poured liquid in real time completely, so as to ensure the removed bone crumbs to be discharged timely. Thus, there is a clear and clean view at the incision and the tissue to be remained is protected from being damaged, which is particularly noticeable in scraping operation (indicated by F4 in Fig. 9). In this way, the surgery risk will be further reduced, and the safety together with the chance of success in surgery will be improved.

In accordance with an embodiment of the present invention, the bore 6 may also be arranged at the spoon bottom, i.e., the convex surface is provided with the bore 6 thereon. In accordance with an embodiment of the present invention, the end portion 1 of the tool bit may have two bores 6 that are located at the spoon center and the spoon bottom respectively. Such design has an advantage that the tool bit 100 with two outlets at the spoon center and spoon bottom may ensure that the poured liquid flows out from both the spoon center and the spoon bottom. In surgery, the spoon center, the spoon bottom, the entire end portion of the tool bit and the tissues that they contact with may be cleaned and cooled by the poured liquid in real time completely, so as to ensure the removed bone crumbs to be discharged timely. Because water flows out from both up and down locations, there is a clear and clean view at the incision and the tissue to be remained is protected more advantageously from being damaged. It is particularly advantageous when the end portion of the tool bit with the spoon-like shape is extended into between the spinal dural and the vertebral body (bone) to be removed and a force is applied in a direction from inside to outside, because the water flowing out from the spoon bottom may protect the spinal dural and the spinal cord below the spoon bottom and the water flowing out from the spoon center may protect the tissue above the spoon center. Further, when one outlet is blocked or jammed in surgery, the other outlet may still allow the liquid to flow out sufficiently to provide the protection. In addition, the arrangement of two outlets allows the liquid to flow to the tissue more sufficiently. As has been discussed above, in this way, the surgery risk will be further reduced, and the safety together with the chance of success in surgery will be further improved.

In accordance with an embodiment of the present invention, the front edge of the end portion 1 of the tool bit is formed with a plurality of tooth slots 7 substantially extending in a direction from a concave surface of the end portion 1 of the tool bit to a convex surface thereof. Each of the tooth slots 7 is substantially at an angle of less than 90 degrees with respect to the direction from the concave surface of the end portion 1 of the tool bit to the convex surface thereof. As illustrated in Fig. 6, each of the tooth slots 7 has an end in communication with the concave surface of the end portion 1 of the tool bit, and has a length L1 reduced gradually from a central part one of the tooth slots to two lateral part ones of the tooth slots in width direction of the end portion of the tool bit. In this way, the teeth with various sizes between adjacent tooth slots 7 may be used to perform the surgery operation, which may be selected easily by the surgery operator.

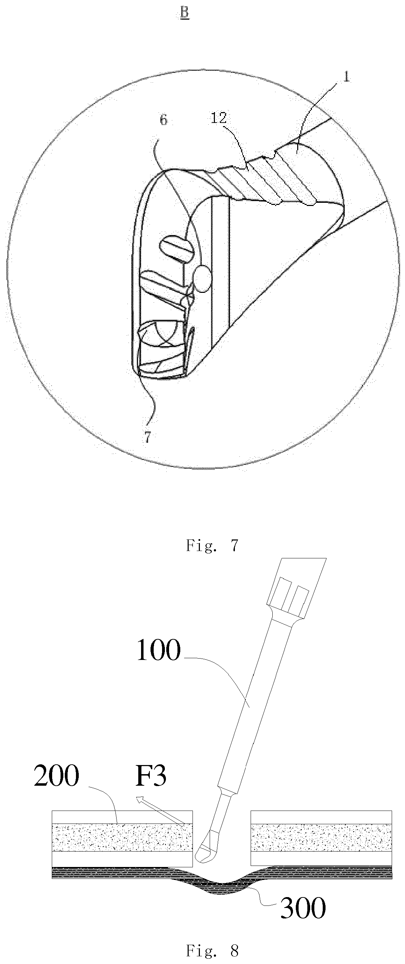

In some embodiments of the present invention, as illustrated in Fig. 7, at least one of two opposed side walls of the end portion 1 of the tool bit connected to the convex surface and the concave surface respectively is provided thereon with a plurality of first grooves 12 spaced from each other. The plurality of first grooves 12 define friction patterns so as to allow the tool bit 100 to have an effect of grinding the bone, and the side wall of the end portion 1 of the tool bit may be used to cut the bone to improve the convenience of use of the tool bit 100. Preferably, each of the first grooves 12 extends in a direction from the spoon center to the spoon bottom and the plurality of first grooves 12 are distributed in a direction from the front to the rear. In this way, the number of the first grooves 12 may be increased to improve the effects of cutting and grinding.

Certainly, the convex surface of the end portion 1 of the tool bit may also be provided with a plurality of second grooves (not shown in figures). The plurality of second grooves define friction patterns to further improve the convenience of use of the tool bit 100. Preferably, each of the second grooves extends in the width direction of the end portion 1 of the tool bit and the plurality of second grooves are distributed in a direction from the front to the rear.

The operation of the tool bit of the ultrasonic osteotome according to an embodiment of the present invention will be described below with reference to Figs. 8-9. When the tool bit 100 having an end portion with the spoon-like shape according to an embodiment of the present invention is operated, on the basis of the principle of using a spoon, any location of the end portion of the tool bit with the spoon-like shape may be used to remove the bone 200. The operator may arbitrarily select any desired manipulation or posture without being limited in operation. It may be used easily. In use, the end portion 1 of the tool bit with the spoon-like shape may extrude into between the spinal dural and the vertebral body (bone) to be removed, and a force may be applied in a direction from inside to outside, as shown in Fig. 8. In this way, the chance of hurting the soft tissues 300 such as spinal cord (marrow) accidently may be reduced. Thus, the surgery risk is reduced and the difficulty of operation is also reduced. In addition, in surgery, by means of cleaning and cooling function of the poured liquid flowing from the bore 6, the spoon center/spoon bottom, the entire end portion 1 of the tool bit and the tissue they contact with may be cleaned and cooled by the poured liquid in real time completely, so as to ensure the bone crumbs (not shown) removed from the bone 200 to be discharged timely. Thus, there is a clear and clean view at the incision and the tissue to be remained is protected from being damaged.

In accordance with an embodiment of the present invention, it further provides an ultrasonic osteotome comprising the tool bit as described in any of the above embodiments. Such design of the ultrasonic osteotome according to the embodiment of the present invention, not only may increase the cutting speed to reduce the time required for surgery, but also can be positioned correctly in cutting operation without skidding to improve the chance of success in surgery.

In the above description, the terms of "an embodiment", "some embodiments", "exemplary embodiment", "example", "specific example" or "some examples" mean that the specific features, structures, materials or characteristics being described in the embodiment or example may be contained in at least one embodiment or example of the present invention. In the description, the exemplary expression for the above terms is not necessarily limited to the same embodiment or example. And the specific features, structures, materials or characteristics described may be combined in any suitable means in any of one or more embodiments or examples.

Although some embodiments of the general inventive concept are illustrated and explained, it would be comprehended by those skilled in the art that the scope of this invention is defined in the appended claims.

## Claims

1. A tool bit (100) of an ultrasonic osteotome, comprising:
an arbor (2); and
an end portion (1) of the tool bit connected to a front end of the arbor (2),
the end portion (1) of the tool bit has a spoon-like shape, wherein the end portion (1) of the tool bit has a toothed front edge;
wherein a front edge of the end portion (1) of the tool bit is formed with a plurality of tooth slots (7) substantially extending in a direction from a concave surface of the end portion (1) of the tool bit to a convex surface thereof; and
wherein each of the tooth slots (7) has an end in communication with the concave surface of the end portion (1) of the tool bit;
**characterized in that**
each of the tooth slots (7) has a length (L1) reduced gradually from a central part one of the tooth slots (7) to two lateral parts ones of the tooth slots (7) in a width direction of the end portion (1) of the tool bit (100).

2. The tool bit (100) according to claim 1, wherein the front edge of the end portion (1) of the tool bit has a maximum width not less than the width of the arbor.

3. The tool bit (100) according to claim 1 or 2, wherein both the arbor and the end portion (1) of the tool bit have hollow inner chambers that are communicated with each other.

4. The tool bit (100) according to claim 3, wherein the end portion (1) of the tool bit is formed with bore (6) which is located in at least one of the spoon center and the spoon bottom of the end portion (1) of the tool bit and is in communication with the hollow inner chambers.

5. The tool bit (100) according to claim 3, further comprising:
a bit body (3) connected to a rear end of the arbor (2),
wherein the arbor (2) and the bit body are of cylindrical shape, the bit body (3) has a cylindrical dimension greater than that of the arbor (2), and the bit body (3) and the arbor (2) are connected via an arc transition.

6. The tool bit (100) according to claim 5, wherein the bit body is provided with a wrench-operation position (5).

7. The tool bit (100) according to claim 6, wherein a threaded connection hole is formed on the side of the bit body (3) opposed to the arbor (2) and is communicated with the hollow inner chambers.

8. The tool bit (100) according to claim 1, wherein each of the tooth slots (7) is at an angle of less than 90 degrees with respect to the direction from the concave surface of the end portion (1) of the tool bit to the convex surface thereof.

9. The tool bit (100) according to claim 1, wherein at least one of two opposed side walls of the end portion (1) of the tool bit connected to the convex surface and the concave surface respectively is provided with a plurality of first grooves (12) spaced from each other.

10. The tool bit (100) according to claim 9 wherein each of the first grooves (12) extends in a direction from a spoon center to a spoon bottom and the plurality of first grooves (12) are distributed in a direction from the front to the rear.

11. The tool bit (100) according to claim 1 or 9, wherein a convex surface of the end portion (1) of the tool bit is provided with a plurality of second grooves.

12. The tool bit (100) according to claim 11, wherein each of the second grooves extends in the width direction of the end portion (1) of the tool bit (100) and the plurality of second grooves are distributed in a direction from the front to the rear.

13. An ultrasonic osteotome comprising the tool bit (100) according to any one of claims 1-12.

## Patentansprüche

1. Werkzeugkopf (100) eines Ultraschallosteotoms, umfassend:
einen Dorn (2); und
einen Endabschnitt (1) des Werkzeugkopfes, der mit einem vorderen Ende des Dorns (2) verbunden ist,
der Endabschnitt (1) des Werkzeugkopfes hat eine löffelartige Form, wobei der Endabschnitt (1) des Werkzeugkopfes eine gezahnte Vorderkante aufweist;
wobei eine Vorderkante des Endabschnitts (1) des Werkzeugkopfes mit einer Vielzahl von Zahnschlitzen (7) ausgebildet ist, die sich im Wesentlichen in einer Richtung von einer konkaven Oberfläche des Endabschnitts (1) des Werkzeugkopfes zu einer konvexen Oberfläche davon erstrecken; und
wobei jeder der Zahnschlitze (7) ein Ende aufweist, das in Verbindung mit der konkaven Oberfläche des Endabschnitts (1) des Werkzeugkopfes ist;
**dadurch gekennzeichnet, dass** jeder der Zahnschlitze (7) eine Länge (L1) aufweist, die allmählich von einem zentralen Schlitz der Zahnschlitze (7) zu zwei seitlichen Schlitzen der Zahnschlitze (7) in einer Breitenrichtung des Endabschnitts (1) des Werkzeugkopfes (100) reduziert wird.

2. Werkzeugkopf (100) nach Anspruch 1, wobei die Vorderkante des Endabschnitts (1) des Werkzeugkopfes eine maximale Breite aufweist, die nicht geringer als die Breite des Dorns ist.

3. Werkzeugkopf (100) nach Anspruch 1 oder 2, wobei sowohl der Dorn als auch der Endabschnitt (1) des Werkzeugkopfes hohle Innenkammern aufweisen, die miteinander in Verbindung stehen.

4. Werkzeugkopf (100) nach Anspruch 3, wobei der Endabschnitt (1) des Werkzeugkopfes mit einer Bohrung (6) ausgebildet ist, die sich in mindestens einer der Löffelmitte und Löffelboden des Endabschnitts (1) des Werkzeugkopfes befindet und mit den hohlen Innenkammern in Verbindung steht.

5. Werkzeugkopf (100) nach Anspruch 3, ferner umfassend:
einen Kopfhauptkörper (3), der mit einem hinteren Ende des Dorns (2) verbunden ist,
wobei der Dorn (2) und der Kopfhauptkörper eine zylindrische Form haben, der Kopfhauptkörper (3) eine zylindrische Abmessung aufweist, die größer als die des Dorns (2) ist, und der Kopfhauptkörper (3) und der Dorn (2) über einen Bogenübergang verbunden sind.

6. Werkzeugkopf (100) nach Anspruch 5, wobei der Kopfhauptkörper mit einem Schlüsselbetätigungsbereich (5) versehen ist.

7. Werkzeugkopf (100) nach Anspruch 6, wobei ein Gewindeverbindungsloch auf der dem Dorn (2) gegenüberliegenden Seite des Kopfhauptkörpers (3) ausgebildet ist und mit den hohlen Innenkammern in Verbindung steht.

8. Werkzeugkopf (100) nach Anspruch 1, wobei jeder der Zahnschlitze (7) in einem Winkel von weniger als 90 Grad in Bezug auf die Richtung von der konkaven Oberfläche des Endabschnitts (1) des Werkzeugkopfes auf die konvexe Oberfläche davon steht.

9. Werkzeugkopf (100) nach Anspruch 1, wobei mindestens eine von zwei gegenüberliegenden Seitenwänden des Endabschnitts (1) des Werkzeugkopfes, der mit der konvexen Oberfläche bzw. der konkaven Oberfläche verbunden ist, mit einer Vielzahl von ersten Nuten (12) versehen ist, die voneinander beabstandet sind.

10. Werkzeugkopf (100) nach Anspruch 9, wobei sich jede der ersten Nuten (12) in einer Richtung von einer Löffelmitte zu einem Löffelboden erstreckt und die Vielzahl von ersten Nuten (12) in einer Richtung von vorne nach hinten verteilt sind.

11. Werkzeugkopf (100) nach Anspruch 1 oder 9, wobei eine konvexe Oberfläche des Endabschnitts (1) des Werkzeugkopfes mit einer Vielzahl von zweiten Nuten versehen ist.

12. Werkzeugkopf (100) nach Anspruch 11, wobei sich jede der zweiten Nuten in der Breitenrichtung des Endabschnitts (1) des Werkzeugkopfes (100) erstreckt und die Vielzahl von zweiten Nuten in einer Richtung von vorne nach hinten verteilt sind.

13. Ultraschallosteotom, umfassend einen Werkzeugkopf (100) nach einem der Ansprüche 1 bis 12.

## Revendications

1. Tête d'outil (100) d'un ostéotome à ultrasons, comprenant:
une tonnelle (2); et
une partie d'extrémité (1) de la tête d'outil reliée à une extrémité avant de la tonnelle (2),
la partie d'extrémité (1) de la tête d'outil a une forme de cuillère, dans laquelle la partie d'extrémité (1) de la tête d'outil a un bord avant denté;
dans laquelle un bord avant de la partie d'extrémité (1) de la tête d'outil est formé avec une pluralité de fentes de dent (7) s'étendant sensiblement dans une direction depuis une surface concave de la partie d'extrémité (1) de la tête d'outil vers une surface convexe de celle-ci; et
dans laquelle chacune des fentes de dent (7) a une extrémité en communication avec la surface concave de la partie d'extrémité (1) de la tête d'outil;
**caractérisée en ce que** chacune des fentes de dent (7) a une longueur (L1) réduite progressivement de l'une centrale des fentes de dent (7) à les deux fentes latérales des fentes de dent (7) dans le sens de la largeur du partie d'extrémité (1) de la tête d'outil (100).

2. Tête d'outil (100) selon la revendication 1, dans laquelle le bord avant de la partie d'extrémité (1) de la tête d'outil a une largeur maximale non inférieure à la largeur de la tonnelle.

3. Tête d'outil (100) selon la revendication 1 ou 2, dans laquelle à la fois la tonnelle et la partie d'extrémité (1) de la tête d'outil ont des chambres internes creuses qui communiquent les uns avec les autres.

4. Tête d'outil (100) selon la revendication 3, dans laquelle la partie d'extrémité (1) de la tête d'outil est formée avec un alésage (6) qui est situé dans au moins l'un parmi le centre de cuillère et le fond de cuillère de la partie d'extrémité (1) de la tête d'outil et qui est en communication avec les chambres internes creuses.

5. Tête d'outil (100) selon la revendication 3, comprenant en outre:
un corps principal de tête (3) relié à une extrémité arrière de la tonnelle (2),
dans laquelle la tonnelle (2) et le corps principal de tête sont de forme cylindrique, le corps principal de tête (3) a une dimension cylindrique supérieure à celle de la tonnelle (2), et le corps principal de tête (3) et la tonnelle (2) sont connectés via une transition d'arc.

6. Tête d'outil (100) selon la revendication 5, dans laquelle le corps principal de tête est pourvu d'une zone d'actionnement par clé (5).

7. Tête d'outil (100) selon la revendication 6, dans laquelle un trou de connexion fileté est formé du côté du corps principal de tête (3) opposé à la tonnelle (2) et est en communication avec les chambres internes creuses.

8. Tête d'outil (100) selon la revendication 1, dans laquelle chacune des fentes de dent (7) est à un angle inférieur à 90 degrés par rapport à la direction à partir de la surface concave de la partie d'extrémité (1) de la tête d'outil vers la surface convexe de celle-ci.

9. Tête d'outil (100) selon la revendication 1, dans laquelle au moins une des deux parois latérales opposées de la partie d'extrémité (1) de la tête d'outil reliées respectivement à la surface convexe et à la surface concave est pourvue d'une pluralité de premières rainures (12) espacées les unes des autres.

10. Tête d'outil (100) selon la revendication 9, dans laquelle chacune des premières rainures (12) s'étend dans une direction allant d'un centre de cuillère à un fond de cuillère et la pluralité de premières rainures (12) sont réparties dans une direction à partir de l'avant vers l'arrière.

11. Tête d'outil (100) selon la revendication 1 ou 9, dans laquelle une surface convexe de la partie d'extrémité (1) de la tête d'outil est pourvue d'une pluralité de secondes rainures.

12. Tête d'outil (100) selon la revendication 11, dans laquelle chacune des secondes rainures s'étend dans le sens de la largeur de la partie d'extrémité (1) de la tête d'outil (100) et la pluralité de secondes rainures sont réparties dans une direction de l'avant vers l'arrière.

13. Ostéotome à ultrasons comprenant une tête d'outil (100) selon l'une quelconque des revendications 1 à 12.
